# EUROPEAN PATENT APPLICATION

(11) **EP 4 647 221 A1**
(43) Date of publication of application: **12.11.2025**
(21) Application number: 24174738.5
(22) Date of filing: 08.05.2024
(51) Int. Cl.: B26B 19/38, A45D 27/46

(54) **PERSONAL CARE SYSTEM COMPRISING A SANITIZING DEVICE AND A CLEANING DEVICE**

(71) Applicant: Koninklijke Philips N.V., 5656 AG Eindhoven (NL)
(72) Inventor: RAO, Ramachandra Ganesh, 5656 AG Eindhoven (NL); GÖZÜTOK, Ahmet, 5656 AG Eindhoven (NL)
(74) Representative: Philips Intellectual Property & Standards

(57) **Abstract**

The present disclosure relates to a personal care system (100) comprising a personal care device (110), a sanitizing device (120), a cleaning device (130), and a processing unit (140). The personal care device includes a treatment unit (112) for treating a user's body. The sanitizing device disinfects the treatment unit using disinfecting light when the treatment unit is in a predefined sanitizing position. The cleaning device cleans the treatment unit using a cleaning-liquid flow when the treatment unit is in a predefined cleaning position. The processing unit receives a first signal indicating a user action in relation to the sanitizing device, and a second signal indicating a degree of debris on/in the treatment unit and/or usage of the treatment unit since a last cleaning of the treatment unit by the cleaning device. The processing unit then generates an output signal relating to a recommended or required cleaning of the treatment unit by the cleaning device based on the received signals.

## Description

### FIELD OF THE INVENTION

The present invention relates to a personal care system comprising a personal care device, a sanitizing device, and a cleaning device.

The invention further relates to a sanitizing device for use in a personal care system.

The invention further relates to a computer-implemented method of operating a personal care system comprising a personal care device, a sanitizing device, and a cleaning device.

### BACKGROUND OF THE INVENTION

Personal care devices, such as electric shavers, are commonly used in daily grooming routines. These devices often come with multiple attachments or treatment units that can be interchanged depending on the user's specific grooming requirements. The treatment units come into direct contact with the user's skin and can accumulate debris over time. This debris can include dead skin cells, hair, or other particulate matter.

Maintaining the hygiene of these treatment units is of utmost concern to ensure the health and safety of the user. Typically, users are responsible for cleaning these treatment units manually, which can be a time-consuming and often overlooked task. Furthermore, manual cleaning may not be sufficient to remove all debris and clean the treatment units effectively.

In addition to cleaning, sanitization is another aspect of maintaining the hygiene of personal care devices. Sanitization involves the use of sanitizing light, such as ultraviolet (UV) light, to kill or inactivate microorganisms that may be present on the treatment units. Similar to cleaning, sanitization is typically a manual process that requires user intervention.

To facilitate the cleaning and sanitizing of a personal care device, it is known to use the personal care device together with a cleaning device, which is configured to clean the treatment unit of the personal care device, or a sanitizing device, which is configured to sanitize the treatment unit of the personal care device. Such a cleaning device is usually configured to clean the treatment unit by means of a cleaning-liquid flow generated by a liquid-displacing member of the cleaning device when the treatment unit is in a predefined cleaning position relative to the cleaning device. Such a sanitizing device is usually configured to disinfect the treatment unit by means of disinfecting light generated by a light source of the sanitizing device when the treatment unit is in a predefined sanitizing position relative to the light source. An example of a cleaning device for an electric shaver is disclosed by EP3737257B1. An example of a sanitizing device for an electric shaver is disclosed by CN214963194U.

The use of personal care devices and their maintenance involves a degree of user interaction and decision-making. For instance, users decide when to clean or sanitize their devices, which can be influenced by factors such as the frequency of use, the type of treatment unit used, and personal hygiene preferences. However, these decisions are often subjective and may not accurately reflect the actual cleanliness or hygiene status of the treatment units. In particular, for a user of a personal care system that comprises, in addition to the personal care device having the treatment unit that treats the user's body, both a sanitizing device and a cleaning device as described here before, it may be difficult to decide when to use the sanitizing device and when to use the cleaning device in order to achieve both an effective sanitizing and an effective cleaning of the treatment unit.

In recent years, there has been a trend towards making personal care devices 'smarter' by incorporating technology that can monitor usage and provide feedback to the user. However, these technologies have primarily focused on improving the performance or functionality of the personal care devices, rather than addressing hygiene or cleanliness concerns.

### SUMMARY OF THE INVENTION

An object of the present invention is to provide a personal care system, comprising a personal care device, a sanitizing device, and a cleaning device, which enables the user to achieve an effective sanitizing and cleaning of the treatment unit of the personal care device.

The invention is defined by the claims.

According to one aspect of the present invention, a personal care system is provided. This system includes a personal care device, a sanitizing device, a cleaning device, and a processing unit. The personal care device comprises a treatment unit configured to treat a portion of a user's body; the sanitizing device is configured to disinfect the treatment unit by means of disinfecting light generated by a light source of the sanitizing device when the treatment unit is in a predefined sanitizing position relative to the light source; and the cleaning device is configured to clean the treatment unit by means of a cleaning-liquid flow generated by a liquid-displacing member of the cleaning device when the treatment unit is in a predefined cleaning position relative to the cleaning device; wherein the processing unit is configured to: receive a first signal generated by the sanitizing device and indicating a user action in relation to the sanitizing device; receive a second signal indicating at least one of: a degree of debris on and/or in the treatment unit; and usage of the treatment unit since a last cleaning of the treatment unit by the cleaning device; when the first signal indicates the user action in relation to the sanitizing device and the second signal indicates that the degree of debris and/or the usage of the treatment unit since the last cleaning exceeds a respective predefined threshold, generate an output signal relating to a recommended or required cleaning of the treatment unit by the cleaning device; and provide the output signal to a feedback member of the personal care system.

The present invention pertains to a personal care system that includes a personal care device, a sanitizing device, a cleaning device, and a processing unit. The personal care device is equipped with a treatment unit that is designed to treat a portion of a user's body. The sanitizing device is configured to disinfect the treatment unit using disinfecting light generated by a light source when the treatment unit is in a predefined sanitizing position relative to the light source. The cleaning device is designed to clean the treatment unit using a cleaning-liquid flow generated by a liquid-displacing member when the treatment unit is in a predefined cleaning position relative to the cleaning device.

The processing unit is a central component of the system, configured to receive a first signal from the sanitizing device which indicates a user action in relation to the sanitizing device. Thereby, the first signal indicates that the user intends to use the sanitizing device. The processing unit further receives a second signal, which indicates the degree of debris on and/or in the treatment unit. Alternatively, or in addition, the second signal may indicate the usage of the treatment unit since a last cleaning of the treatment unit by the cleaning device. When the second signal indicates that the degree of debris and/or the usage of the treatment unit since the last cleaning exceeds a respective predefined threshold, the treatment unit is considered to be insufficiently clean for an efficient sanitizing process by means of the sanitizing device. Accordingly, when the first signal indicates an intended use of the sanitizing device and the second signal indicates that the treatment unit is insufficiently clean for proceeding with the sanitizing process, the processing unit provides feedback to the user regarding a required or recommended cleaning of the treatment unit before being sanitized by the sanitizing device. This configuration allows for a more personalized and efficient cleaning and sanitizing process, enhancing the user's personal care routine.

Thus, the processing unit is designed to generate an output signal relating to a recommended or mandatory cleaning of the treatment unit by the cleaning device when the first signal indicates a user action in relation to the sanitizing device and the second signal indicates that the degree of debris and/or the usage of the treatment unit since the last cleaning exceeds a predefined threshold. This feature provides the user with timely and accurate feedback on a required or recommended cleaning of the treatment unit before an intended sanitization of the treatment unit, thereby ensuring a more effective and hygienic treatment. In other words, the user is recommended to first clean and then to sanitize the treatment unit, and not the other way around. This is because, for example, a shaver with debris of skin/cells/hair will (very likely) provide an environment that encourages bacterial growth. UV light or other types of sanitizing light kill micro-organisms by damaging their DNA or RNA, crippling their ability to make copies of themselves. However, UV light (or other types of sanitizing light) does not penetrate most solid substances or unclear fluids. It will therefore not penetrate the debris to reach micro-organisms beneath the debris particles. Ideally, the personal care device of the present example therefore needs to be pre-cleaned (to remove debris) and then sanitized for an optimal sanitizing efficiency, i.e., a maximum micro-organism inactivation efficacy.

The proposed personal care system leverages a combination of sanitization, cleaning, and processing unit(s) to maintain the hygiene and cleanliness of personal care devices. The system provides an improved cleaning and sanitizing process, enhancing the user's personal care routine. In particular, the system provides timely and accurate feedback on a required or recommended cleaning of the treatment unit by means of the cleaning device (in case the user intends to sanitize the treatment unit by means of the sanitizing device), ensuring a more effective overall hygienic treatment. In an embodiment, the system's ability to store and utilize usage and cleaning data relating to the treatment unit allows for a more accurate assessment of the cleanliness of the treatment unit when the user intends to sanitize the treatment unit, leading to more effective cleaning and sanitization.

In some embodiments, the first signal may indicate at least one of: placement of the treatment unit in the predefined sanitizing position by the user; and activation of the sanitizing device by the user. Placement of the treatment unit in the sanitizing device and/or activation of the sanitizing device by the user are practical examples of a measurable intention of the user to use the sanitizing device. Thereby, by using the first signal, the processing unit is able to determine in a reliable manner whether the user intends to use the sanitizing device for sanitizing the treatment unit, and to trigger the feedback about a required or recommended cleaning of the treatment unit in case the second signal indicated that the treatment unit is insufficiently clean.

In yet another embodiment, the processing unit may prevent the activation of the light source of the sanitizing device when the first signal indicates a user action in relation to the sanitizing device and the second signal indicates that the degree of debris and/or the usage of the treatment unit since the last cleaning exceeds the respective predefined threshold. This feature ensures that the light source of the sanitizing device is not activated and, as a result, the sanitizing process cannot be started when the treatment unit is determined to be insufficiently clean when the user intends to use the sanitizing device, thereby preventing ineffective use of the sanitizing device.

In some embodiments, the second signal may be provided by a sensor which is arranged in the personal care device and configured to measure a parameter which is related to the degree of debris on and/or in the treatment unit. The inclusion of said sensor in the personal care device allows for real-time monitoring of the degree of debris or the cleanliness of the treatment unit, and thus enables accurate and timely feedback to the processing unit, via the second signal, about the cleanliness condition of the treatment unit. This ensures an accurate triggering by the processing unit of the feedback unit about a required or recommended pre-cleaning of the treatment unit when the user intends to sanitize the treatment unit, based on the actual cleanliness condition of the treatment unit measured by the sensor, thereby optimizing the maintenance routine for the treatment unit.

In some embodiments, the personal care system may include a data storage unit configured to store usage data about the treatment unit received from the personal care device and cleaning data about the treatment unit received from the cleaning device and/or received from the personal care device. In this embodiment, the second signal may indicate usage of the treatment unit since the last cleaning of the treatment unit and may be provided by the data storage unit based on the stored usage data and cleaning data. In this embodiment, the second signal, which indicates the cleanliness condition of the treatment unit, is based on detected usage of the treatment unit since it was last cleaned by the cleaning device. Said usage may be determined by the processing unit based on the usage data about the treatment unit and the cleaning data about the treatment unit stored in the data storage unit. When the processing unit determines that said usage exceeds a predefined usage threshold, the processing unit determines that the cleanliness condition of the treatment unit is insufficient for an effective sanitizing process. In this way, the cleanliness condition of the treatment unit may be estimated without the use of a dedicated sensor in the personal care device for detecting the degree of debris. For example, the second signal may indicate, with respect to the treatment unit, the usage since the last cleaning of the treatment unit by the cleaning device as an accumulated treatment time and/or a number of treatment sessions performed since the last cleaning of the treatment unit by the cleaning device.

In some embodiments, the data storage unit may be provided in the personal care device. Having the data storage unit within the personal care device enhances data collection and reliability, as in practical cases the stored usage data about the treatment unit may be generated by and received from one or more sensors in the personal care device, and the stored cleaning data about the treatment unit may be generated and received while the personal care device is in the predefined cleaning position relative to the cleaning device.

In other embodiments, the personal care device may include a main body and at least two different treatment units that can each be selectively coupled to the main body. The personal care device may further include an identification system configured to identify which of the different treatment units is actually coupled to the main body. The data storage unit may be configured to store received usage data and cleaning data for each of the different treatment units. The processing unit may be configured to: receive an identification signal from the sanitizing device indicating which of the different treatment units is identified in the sanitizing position; and receive, from the data storage unit and for the identified treatment unit, the second signal indicating the usage of the identified treatment unit since a last cleaning of the identified treatment unit by the cleaning device. This feature allows for personalized cleaning and sanitizing schedules for the different treatment units of the personal care device, enhancing the overall effectiveness of the personal care system. In particular, the identification signal received by the processing unit enables the processing unit to receive, from the data storage unit and amongst the usage data for all different treatment units, the correct usage data for the treatment unit which is actually placed in the sanitizing device.

In some embodiments, the sanitizing device may be configured to receive the personal care device including the main body and the treatment unit coupled to the main body, and to generate the identification signal based on an output signal provided by the identification system of the personal care device. In this embodiment, the identification signal received by the processing unit from the sanitizing device and indicating which of the different treatment units is identified in the sanitizing position, is provided by the identification system of the personal care device when the personal care device is received by the sanitizing device. In this way, the sanitizing device does not need to be provided with its own identification system to identify which of the different treatment units is placed in the sanitizing device.

In another aspect of the invention, there is provided a sanitizing device for use in a personal care system comprising the sanitizing device, a personal care device, and a cleaning device, wherein the personal care device comprises a treatment unit configured to treat a portion of a user's body, and wherein the cleaning device is configured to clean the treatment unit by means of a cleaning-liquid flow generated by a liquid-displacing member of the cleaning device when the treatment unit is in a predefined cleaning position relative to the cleaning device; wherein the sanitizing device is configured to disinfect the treatment unit and comprises: a light source configured to generate disinfecting light; a holding member configured to hold the treatment unit in a predefined sanitizing position relative to the light source such that the treatment unit is exposable to the disinfecting light; and a processing unit; wherein the processing unit is configured to: receive a first signal indicating a user action in relation to the sanitizing device; receive a second signal indicating at least one of: a degree of debris on and/or in the treatment unit; and usage of the treatment unit since a last cleaning of the treatment unit by the cleaning device; when the first signal indicates the user action in relation to the sanitizing device and the second signal indicates that the degree of debris and/or the usage of the treatment unit since the last cleaning exceeds a respective predefined threshold, generate an output signal relating to a recommended or required cleaning of the treatment unit by the cleaning device; and provide the output signal to a feedback member of the personal care system.

The sanitizing device according to this aspect of the invention may be part of a personal care system according to the invention as described here before and, as such, comprises the processing unit of the personal care system. In alternative embodiments of the personal care system according to the invention, the processing unit may be part of the personal care device or part of a remote device, such as a smart phone.

In some embodiments, the sanitizing device may comprise the feedback member. This may provide a convenient place for a user to receive a notification about a required or recommended cleaning of the treatment unit by the cleaning device in case the user intends to use the sanitizing device to sanitize the treatment unit.

In some embodiments, the sanitizing device may further comprise a detector configured to detect placement of the treatment unit in the predefined sanitizing position by the user, wherein the first signal is provided by the detector; and/or a user-operable input element by which a user is enabled to activate the sanitizing device, wherein the first signal is provided by the user-operable input element. These may provide efficient and/or effective ways of providing the first signal indicating whether a user intends to use the sanitizing device.

In some embodiments, the sanitizing device may further comprise a data storage unit configured to receive usage data about the treatment unit from the personal care device and cleaning data about the treatment unit from the cleaning device and/or the personal care device, and to store the received usage data and the received cleaning data, wherein the second signal indicates usage of the treatment unit since the last cleaning of the treatment unit by the cleaning device and is provided by the data storage unit based on the stored usage data and cleaning data.

In some embodiments, the personal care device of the personal care system may comprise a main body and at least two different treatment units that can each be selectively coupled to the main body, and the data storage unit may be configured to store, for each of the different treatment units, received usage data and received cleaning data; and wherein the processing unit may be configured to: receive an identification signal indicating which of the different treatment units is identified in the sanitizing position; and receive, from the data storage unit and for the identified treatment unit, the second signal indicating the usage of the identified treatment unit since a last cleaning of the identified treatment unit by the cleaning device.

In some embodiments, the sanitizing device may be configured to: receive the personal care device including the main body and the treatment unit coupled to the main body; and generate the identification signal based on an output signal provided by an identification system of the personal care device which is configured to identify which of the different treatment units is actually coupled to the main body.

In yet another aspect of the invention, there is provided a computer-implemented method of operating a personal care system comprising a personal care device, a sanitizing device, and a cleaning device, wherein: the personal care device comprises a treatment unit configured to treat a portion of a user's body; the sanitizing device is configured to disinfect the treatment unit by means of disinfecting light generated by a light source of the sanitizing device when the treatment unit is in a predefined sanitizing position relative to the light source; and the cleaning device is configured to clean the treatment unit by means of a cleaning-liquid flow generated by a liquid-displacing member of the cleaning device when the treatment unit is in a predefined cleaning position relative to the cleaning device; wherein the method comprises: receiving a first signal generated by the sanitizing device and indicating a user action in relation to the sanitizing device; receiving a second signal indicating at least one of: a degree of debris on and/or in the treatment unit; and usage of the treatment unit since a last cleaning of the treatment unit by the cleaning device; when the first signal indicates the user action in relation to the sanitizing device and the second signal indicates that the degree of debris and/or the usage of the treatment unit since the last cleaning exceeds a respective predefined threshold, generating an output signal relating to a recommended or required cleaning of the treatment unit by the cleaning device; and providing the output signal to a feedback member of the personal care system.

In another aspect of the invention, there is provided a computer program comprising computer program code which is configured, when said program is run on a processing unit of a personal care system, to implement the method according to the invention.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying figures, in which:
Fig. 1 depicts a block diagram of a personal care system, according to aspects of the present invention;
Fig. 2 depicts a block diagram of a personal care system, according to aspects of the present invention;
Fig. 3 depicts a block diagram of a personal care system with multiple treatment units, according to aspects of the present invention;
Fig. 4 depicts a block diagram of a sanitizing device, according to aspects of the present invention;
Fig. 5 depicts a block diagram of a sanitizing device, according to aspects of the present invention;
Fig. 6 depicts a flow diagram of a computer-implemented method of operating a personal care system, according to aspects of the present invention; and
Fig. 7 depicts an example of a computer within which one or more parts of an embodiment may be employed.

### DETAILED DESCRIPTION OF THE EMBODIMENTS

The invention will be described with reference to the Figures.

It should be understood that the Figures are merely schematic and are not drawn to scale. It should also be understood that, when same reference numerals are used throughout the Figures, they intend to indicate the same or similar parts.

It should also be understood that the detailed description and specific examples, while indicating exemplary embodiments of the systems, devices and methods, are intended for purposes of illustration only and are not intended to limit the scope of the invention. These and other features, aspects, and advantages of the systems, devices and methods of the present invention will become better understood from the following description, appended claims, and accompanying figures. The mere fact that certain measures are recited in mutually different dependent claims does not indicate that a combination of these measures cannot be used to advantage.

The present invention pertains to a personal care system that includes a personal care device, a sanitizing device, a cleaning device, and a processing unit. The personal care device is equipped with a treatment unit that is configured to treat a portion of a user's body. The sanitizing device is configured to disinfect the treatment unit using disinfecting light generated by a light source of the sanitizing device when the treatment unit is in a predefined sanitizing position relative to the light source. The cleaning device is configured to clean the treatment unit using a cleaning-liquid flow generated by a liquid-displacing member of the cleaning device when the treatment unit is in a predefined cleaning position relative to the cleaning device.

The proposed personal care system leverages a combination of sanitization, cleaning, and processing unit(s) to maintain the hygiene and cleanliness of personal care devices. The system provides an improved cleaning and sanitizing process, enhancing the user's personal care routine. In particular, the system provides timely and accurate feedback on a required or recommended cleaning of the treatment unit by means of the cleaning device in case the user intends to sanitize the treatment unit by means of the sanitizing device and when, in that case, the treatment unit is determined to be insufficiently clean for enabling an efficient disinfecting process by means of the sanitizing device. Said feedback will ensure a more effective overall hygienic treatment. In an embodiment, the system's ability to store and utilize usage and cleaning data relating to the treatment unit allows for a more accurate assessment of the cleanliness of the treatment unit at the moment when the user intends to sanitize the treatment unit, leading to more effective cleaning and sanitization.

For the avoidance of doubt, cleaning is to be understood as reducing an amount of debris on and/or in an object (e.g., the treatment unit), e.g., by means of a flow of water or another cleaning liquid, whereas sanitizing is to be understood as killing or inactivating, by means of disinfecting light, microorganisms on and/or in an object (e.g., the treatment unit). In other words, cleaning and sanitization are distinct yet complementary processes in maintaining cleanliness and hygiene. Cleaning involves the removal of dirt, debris, and impurities from surfaces through the use of, for example, detergents, water, and mechanical action like scrubbing or wiping. It primarily targets the physical removal of contaminants. Sanitization, on the other hand, goes a step further by reducing the number of harmful microorganisms to safe levels, minimizing the risk of infection or illness. It typically involves the application of UV light, or another type of disinfecting light such as blue light, to kill micro-organisms such as bacteria, viruses, and other pathogens. In summary, cleaning focuses on the physical removal of visible dirt, debris and/or impurities, whereas sanitization reduces/minimizes microbial contamination, ensuring a hygienic environment.

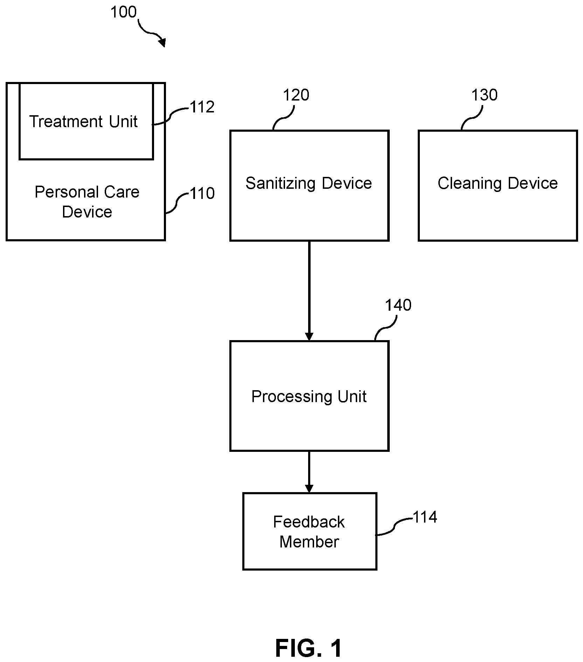

Referring to Fig. 1, a block diagram of an embodiment of a personal care system 100 according to an aspect of the present invention is depicted. The personal care system 100 includes a personal care device 110, a sanitizing device 120, a cleaning device 130, a processing unit 140, and a feedback member 114.

The personal care device 110 comprises a treatment unit 112 configured to treat a portion of a user's body. The personal care device 110 can comprise, among other things which the skilled person would appreciate, an electric shaver, an electric toothbrush, an oral cleaning device, an interdental device, a haircare device, a hair removal device, a skincare device, a body grooming device, an epilator device, an intense pulse light (IPL) device, a body trimming device, a beauty device, an exfoliator, etc., and thus the treatment unit 112 can comprise, among other things, a shaver head, a toothbrush head, a treatment portion of a beauty device or exfoliator, etc. Such personal care devices and treatment units thereof are well known to the skilled person and will not be described in detail.

The sanitizing device 120 is configured to disinfect the treatment unit 112. The sanitizing device 120 generates disinfecting light from a light source when the treatment unit 112 is in a predefined sanitizing position relative to the light source of the sanitizing device 120. Such sanitizing devices are also well known to the skilled person and do not need to be described in much detail. They may comprise a casing having a receiving space to receive the personal care device in the predefined sanitizing position relative to the light source, which is also arranged in the casing. The casing may be openable and closeable by means of a cover member to ensure that, during the disinfection process, the disinfecting light may not escape from the casing and injure the user's eyes.

The cleaning device 130 is configured to clean the treatment unit 112. The cleaning device 130 generates a cleaning-liquid flow from a liquid-displacing member when the treatment unit 112 is in a predefined cleaning position relative to the cleaning device 130. It should be noted that the treatment unit 112 can be placed in the predefined cleaning position alone or while still attached to the personal care device 110. Such cleaning devices are also well known to the skilled person and will not be described in detail. An example of a cleaning device for an electric shaver is disclosed by EP3737257B1 in the name of the applicant.

The processing unit 140 is configured to receive and process signals. A first signal received by the processing unit 140 is generated by the sanitizing device 120 and indicates a user action in relation to the sanitizing device 120. A second signal received by the processing unit 140 indicates at least one of: a degree of debris on and/or in the treatment unit 112; and usage of the treatment unit 112 since a last cleaning of the treatment unit 112 by the cleaning device 130. Debris on and/or in the treatment unit 112 can alternatively be understood as detritus/dirt/pollution on and/or in the treatment unit.

The processing unit 140 generates an output signal relating to a recommended or required cleaning of the treatment unit 112 by the cleaning device 130 when: the second signal indicates that the degree of debris and/or the usage of the treatment unit 112 since the last cleaning exceeds a respective predefined threshold; and the first signal indicates the user action in relation to the sanitizing device 120.

The processing unit 140 provides the output signal to the feedback member 114. For example, the feedback member 114 can comprise a display or a light or a vibrating actuator, etc., capable of indicating to a user that it is recommended or required that they clean the treatment unit 112 with the cleaning device 130 first before they use the sanitizing device 120. In this embodiment, the feedback member 114 is provided independently, i.e., external to any of the other devices or units of the personal care system 100 (for example, in a user's smart device, such as a smartphone or tablet). In other embodiments, however, the feedback member 114 can be directly provided in one or more of the personal care device 110 or the sanitizing device 120 or the cleaning device 130.

The first signal indicating a user action in relation to the sanitizing device 120 is to be understood as a signal indicating a user's intention to use the sanitizing device 120. In this embodiment, the first signal indicates at least one of: placement of the treatment unit 112 in the predefined sanitizing position by the user; and activation of the sanitizing device 120 by the user. Placement of the treatment unit 112 in the sanitizing device 120 and/or activation of the sanitizing device 120 by the user are practical examples of a measurable intention of the user to use the sanitizing device 120. Thereby, by using the first signal, the processing unit 140 is able to determine in a reliable manner whether the user intends to use the sanitizing device 120 for sanitizing the treatment unit 112, and to trigger the feedback about a required or recommended cleaning of the treatment unit 112 when at the same time the second signal indicates that the treatment unit 112 is insufficiently clean. In other embodiments, the first signal can indicate any other suitable user action in relation to the sanitizing device 120, as would be understood by the skilled person.

It should be noted that placement of the treatment unit 112 in the predefined sanitizing position can be understood as the user placing the treatment unit 112 alone in the predefined sanitizing position (i.e., in the sanitizing device 120) or the user placing the entire (or a portion of the) personal care device 110 including the treatment unit 112 in the predefined sanitizing position (i.e., in the sanitizing device 120). For instance, in embodiments in which the sanitizing device 120 comprises a casing with a receiving space for the treatment unit 112 as described herein before, placing the treatment unit 112 in the predefined sanitizing position may comprise placing the entire personal care device 110 including the treatment unit 112 within the receiving space of the casing.

In some embodiments, the sanitizing device 120 can comprise a detector configured to detect placement of the treatment unit 112 in the predefined sanitizing position by the user, wherein the first signal can thus be provided by the detector. For example, the detector can comprise at least one of: an optical sensor; a proximity sensor; and a physical/mechanical switch. In some embodiments, the sanitizing device 120 can also/instead comprise a user-operable input element (e.g., a button, a touch element, a switch, etc.) by which a user is enabled to activate the sanitizing device 120, wherein the first signal can thus also/alternatively be provided by the user-operable input element.

In some embodiments, the second signal (received by the processing unit 140) is generated by the personal care device 110 indicating usage of the treatment unit 112 since a last cleaning of the treatment unit 112 by the cleaning device 130. For example, the personal care device 110 can track when the treatment unit 112 is cleaned by the cleaning device 130 (or be provided with this information by the cleaning device 130 itself). This information can then be combined with usage information of the treatment unit 112 to determined usage of the treatment unit 112 since a last cleaning of the treatment unit 112 by the cleaning device 130. In other embodiments, however, the second signal indicates a degree of debris on and/or in the treatment unit 112 and can be generated by the personal care device 110, the sanitizing device 120, or the cleaning device 130. More details as to these embodiments will be discussed below.

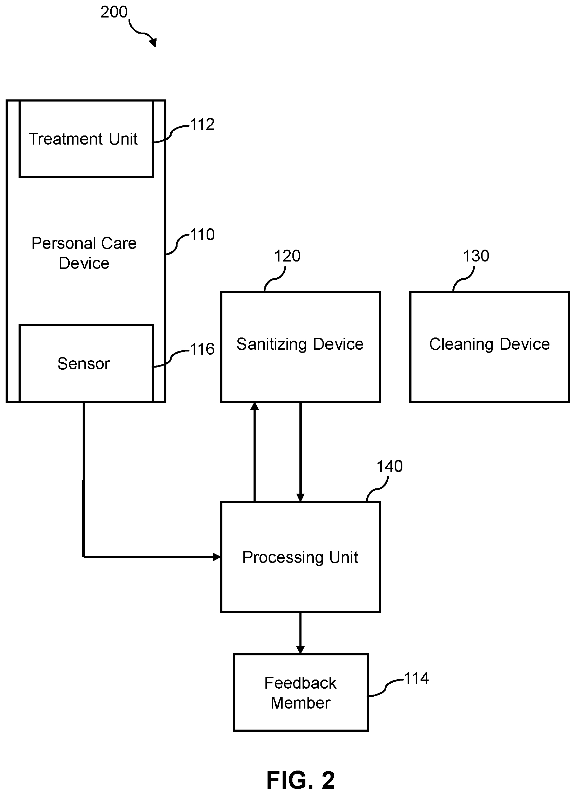

Referring now to Fig. 2, a block diagram of an embodiment of a personal care system 200 according to an aspect of the present invention is depicted. The personal care system 200 is substantially similar to the personal care system 100 of Fig. 1. However, their differences will now be described.

Within the personal care system 200, the personal care device 110 further comprises a sensor 116. The sensor 116 is arranged in the personal care device 110 and configured to measure a parameter which is related to the degree of debris on and/or in the treatment unit 112. For example, the sensor 116 can comprise at least one of: an optical sensor (e.g., to optically detect the presence of debris); an acoustic sensor (e.g., to detect changes in sound patterns caused by the presence of debris); a weight sensor (e.g., to monitor the weight of the treatment unit and thus to detect an increase of weight due to debris); a pressure sensor (e.g., to monitor pressure on the treatment unit and thus to detect an increase of pressure on the treatment unit due to debris); and a vibration sensor (which, for example, can detect abnormal vibrations during use of the personal care device caused by debris, e.g., by calculating a difference in a detected frequency from the default natural frequency/frequencies of the personal care device 110). That is, in this embodiment, the second signal indicates an actual degree of debris on and/or in the treatment unit 112 and is provided by the sensor 116 provisioned in the personal care device 110.

The inclusion of said sensor 116 in the personal care device 110 allows for real-time monitoring of the degree of debris or the cleanliness of the treatment unit 112, and enables accurate and timely feedback to the processing unit 140, via the second signal, about the cleanliness condition of the treatment unit 112. This ensures an accurate triggering by the processing unit 140 of the feedback member 114 about a required or recommended pre-cleaning of the treatment unit 112, when the first signal indicates that the user intends to sanitize the treatment unit 112, based on the actual cleanliness condition of the treatment unit 112 measured by the sensor 116, thereby optimizing the maintenance routine for the treatment unit 112.

In other embodiments, however, the sensor 116 can be provided in the sanitizing device 120, such that the parameter related to the degree of debris on and/or in the treatment unit 112 can be measured when the treatment unit 112 is placed in the sanitizing device 120 (e.g., in the predefined sanitizing position).

In this embodiment, the processing unit 140 is further configured to, when the first signal indicates the user action in relation to the sanitizing device 120 and the second signal indicates that the degree of debris and/or the usage of the treatment unit 112 since the last cleaning exceeds the respective predefined threshold, prevent the activation of the light source of the sanitizing device 120. This feature ensures that the light source of the sanitizing device 120 is not activated and, as a result, the sanitizing process cannot be started when the treatment unit 112 is determined to be insufficiently clean in case the user intends to use the sanitizing device 120, thereby preventing ineffective use of the sanitizing device 120. On the other hand, if the second signal indicates that a degree of debris and/or usage of the treatment unit 112 since the last cleaning is below the respective predefined threshold, then the processing unit 140 can trigger the activation of the light source of the sanitizing device 120 or simply not prevent the activation.

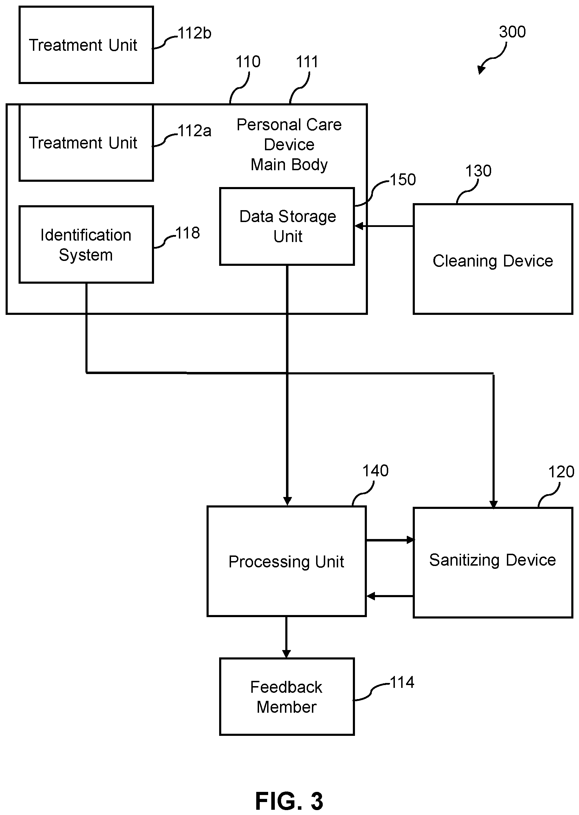

Referring now to Fig. 3, a block diagram of an embodiment of a personal care system 300 according to an aspect of the present invention is depicted. The personal care system 300 is substantially similar to the personal care systems 100 and 200 of Figs. 1 and 2 respectively. However, their differences will now be described.

In this embodiment, the personal care system 300 comprises a data storage unit 150. In this embodiment, the data storage unit 150 is provided in the personal care device 110, but in other embodiments it can be provided in other components of the system 300 or even on its own, i.e., independently. Having the data storage unit 150 within the personal care device 110 enhances data collection and reliability, as in practical cases stored usage data about the treatment unit, 112a and 112b, can be generated by and received from one or more sensors in the personal care device 110, and stored cleaning data about the treatment unit, 112a and 112b, can be generated and received while the personal care device 110 is in the predefined cleaning position relative to the cleaning device 130. The data storage unit 150 is configured to receive usage data about the treatment unit, 112a and 112b, from the personal care device 110 and cleaning data about the treatment unit, 112a and 112b, from the cleaning device 130 and/or the personal care device 110. The data storage unit 150 is further configured to store the received usage data and the received cleaning data. In this embodiment, the second signal indicates usage of the treatment unit 112a since the last cleaning of the treatment unit 112a by the cleaning device 130 and is provided by the data storage unit 150 based on the stored usage data and cleaning data.

In this embodiment, the personal care device 110 comprises a main body 111 and two different treatment units, 112a (in Fig. 3 attached to the main body 111) and 112b (in Fig. 3 unattached / attachable to the main body 111 instead of the treatment unit 112a), that can each be selectively coupled to the main body 111. In other embodiments, there can, of course, be more than two different treatment units or even just one. Such personal care devices with two or more treatment units, that can be alternatively coupled to a main body of the personal care device, are well known to the skilled person and do not need to be described in detail. An electric shaver may, e.g., be provided with a shaving unit that is releasably coupled to the main body of the shaver and replaceable by, e.g., a nose trimming unit, a facial cleansing brush, or a precision trimmer. Each different treatment unit may be driven by a motor accommodated in the main body when the treatment unit is coupled to the main body, as is well known to the skilled person.

The personal care device 110 also comprises an identification system 118 configured to identify which of the different treatment units, 112a or 112b, is actually coupled to the main body 111. The use of an identification system 118 allows for personalized cleaning and sanitizing schedules for the different treatment units, 112a and 112b, of the personal care device 110, enhancing the overall effectiveness of the personal care system 300. In particular, the identification system 118 is configured to generate an identification signal indicating which of the different treatment units, 112a, 112b, is actually coupled to the main body 111. The identification signal may be received by the processing unit 140. Based on the identification signal, the processing unit 140 is enabled to update, during use of the personal care device 110 with the treatment unit 112a or 112b and during cleaning of the treatment unit 112a or 112b, the usage data and the cleaning data stored in the data storage unit 150 for the treatment unit 112a or 112b actually coupled to the main body 111. Based on the identification signal, the processing unit 140 is also enabled to receive, from the data storage unit 150 and amongst the usage data for all different treatment units, 112a and 112b, the correct usage data for the treatment unit 112a which is actually placed in the sanitizing device 120. For example, as the skilled person would understand, the identification system 118 can comprise at least one of: an RFID system (e.g., wherein each treatment unit, 112a and 112b, is equipped with a different RFID tag/label, and an RFID reader in the main body 111 of the personal care device 110 can be configured to read the RFID tag of the treatment unit 112a attached to the main body 111); a barcode / QR-code system (e.g., wherein each treatment unit, 112a and 112b, is equipped with a different barcode or QR-code, and a scanner device in the main body 111 of the personal care device 110 can be configured to read the barcode or QR-code of the treatment unit 112a attached to the main body 111); and a NFC system (e.g., wherein each treatment unit, 112a and 112b, is equipped with a different NFC tag, and a NFC reader in the main body 111 of the personal care device 110 can be configured to read the NFC tag of the treatment unit 112a attached to the main body 111). As the skilled person would understand, however, any suitable identification system could be used for this purpose. For example, EP3855976B1 in the name of the applicant discloses an identification system in an electric shaver, wherein the type of treatment unit actually coupled to the main body of the shaver is determined based on the measured electrical current in the shaver motor and the measured vibrations of the main body caused by driving the treatment unit.

In the embodiment shown in Fig. 3, the data storage unit 150 is therefore configured to store, for each of the different treatment units, 112a and 112b, received usage data and received cleaning data. Further, in some embodiments, the processing unit 140 can be configured to receive an identification signal from the sanitizing device 120 indicating which of the different treatment units, 112a or 112b, is identified in the sanitizing position, Based on the received identification signal, the processing unit 140 may request and receive, from the data storage unit 150 and for the identified treatment unit 112a represented by the identification signal, the second signal indicating the usage of the identified treatment unit 112a since a last cleaning of the identified treatment unit 112a by the cleaning device 130.

In some embodiments, the second signal can indicate, with respect to (each) treatment unit, 112a and 112b, an accumulated treatment time and/or a number of treatment sessions performed since the last cleaning of the treatment unit, 112a or 112b, by the cleaning device 130.

Further, in the embodiment of Fig. 3, the sanitizing device 120 is configured to receive the personal care device 110, including the main body 111, the identification system 118 and the treatment unit 112a coupled to the main body 111, and to generate the identification signal based on an output signal provided by the identification system 118 of the personal care device 110. In this way, the sanitizing device 120 does not need to be provided with its own identification system to identify which of the different treatment units, 112a or 112b, is placed in the sanitizing device 120. This, of course, is not essential to the working of this embodiment, as the skilled person would understand. When the first signal indicates a user action in relation to the sanitizing device 120, as described here before with respect to the embodiment of Fig. 1, and the second signal indicates that the usage of the identified treatment unit 112a, since the last cleaning of the identified treatment unit 112a by means of the cleaning device 130, exceeds the predefined threshold, the processing unit 140 generates an output signal relating to a recommended or required cleaning of the identified treatment unit 112a by the cleaning device 130 and provides the output signal to the feedback member 114.

In an example implementation of the proposed concept(s), an embodiment can be used to provide operation modes for a self-aware electric shaver system. For instance, the system can check whether the shaver is sufficiently clean when the user wants to sanitize it. When the shaver is not sufficiently clean, the user can then be instructed to clean the shaver first using a cleaning device. This can be executed via a computer-implemented method that is executed (either by the shaver, the sanitizing device, or in an app/cloud) when the shaver is placed in the sanitizing device, for example.

For instance, the decision to recommend cleaning of the shaver can be based on one or more of the following: a number of shaving sessions performed since a last cleaning or sanitization; a number of shaving sessions performed and weighted with an average increase or deviation of a measured shaver motor torque relative to a predefined threshold; and frequency domain setpoints and/or threshold-based decisions. In these examples, the current of the shaver motor can be used to detect motor torque, and thus, a motor current measurement can be made as being correlated to slow (e.g., long-term) torque changes caused by dirt within the shaving system. An example of such a system is described in EP 3157716B1 in the name of the applicant. Further, setpoints or thresholds (e.g., a lookup table or preset frequency ranges) of a measured motor current value (corresponding to a shaver torque value) can be stored and used as an input to initiate a notification to the user to clean the shaver. For example, if a measured motor current value is above a predetermined threshold, then this can indicate that the shaver is dirty, and thus a notification can be output to the user to clean the shaver. The values of the stored motor current and their corresponding torque can be in the natural or the frequency domain.

Other data that can also be used to make the decision whether to recommend or instruct the user to clean the shaver prior to sanitizing the shaver can include one or more of: treatment number; treatment duration; treatment time stamp; treatment unit ID; average time of skin/tooth/gum contact. Furthermore, the attributes of 'user settings' or `personalized settings' can include user choices of storing pre-defined session data in an app or cloud, and can describe personal preferences for frequency of sanitization cycles per routine or per treatment unit, switching on/off for connection protocols between the personal care device, an app and network at preset intervals, and choices on receiving data transfers or device notifications. Some of these personal preferences, when adapted beyond the default settings for example, may enable faster determination of the cleaning status by the system. Storage means may be provided by a memory in the personal care device, in a connected (mobile) device or in the cloud.

In another exemplary embodiment, a user can place a shaver in a sanitizing UV case (i.e., a sanitizing device). If there is an active cleaning reminder, the user can immediately be warned to clean the device before starting the sanitization process. If there is no active cleaning reminder, then the user can sanitize the shaver (but if it has not been used since the last sanitization, the user can be warned that sanitization is not necessary).

In another example, after a shaving session, a user can be provided with a cleaning reminder. If the user cleans the shaver, the cleaning reminder will be removed. However, if they do not clean the shaver, the cleaning reminder will remain active, and if the user then goes to sanitize the shaver, they will be warned to clean the shaver before starting the sanitization process. The disinfecting light may even be disabled until the user has cleaned the shaver.

As mentioned above, the processing of data and decision making can take place in the personal care device or the sanitizing device, or it can take place on a user's smart device (e.g., smartphone or tablet) or it can take place in the cloud. For example, the user can then receive a notification to clean and/or sanitize their personal care device, e.g., their shaver, either through their own smart device or through an indicator/display on the personal care device itself.

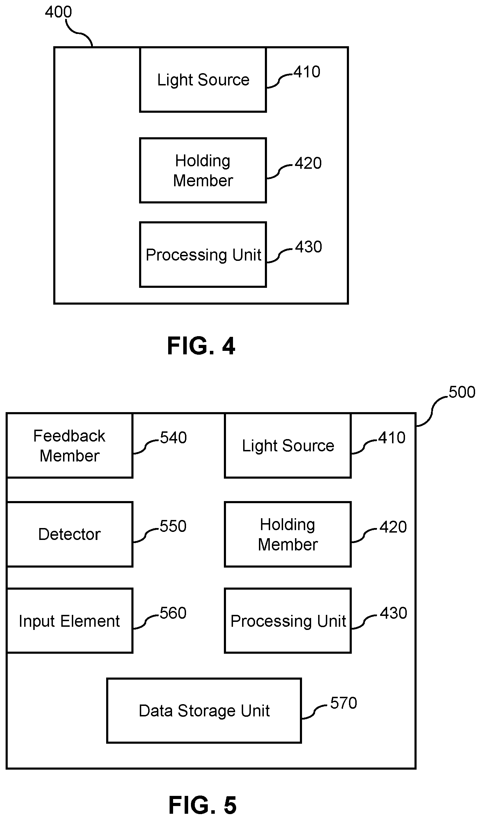

Referring now to Fig. 4, a block diagram of an embodiment of a sanitizing device 400 according to an aspect of the present invention is depicted. The sanitizing device 400 is for use in a personal care system (such as have been described above, e.g., personal care systems 100, 200, and 300 described in Figs. 1, 2 and 3, respectively) comprising the sanitizing device 400, a personal care device, and a cleaning device, wherein the personal care device comprises a treatment unit configured to treat a portion of a user's body, and wherein the cleaning device is configured to clean the treatment unit by means of a cleaning-liquid flow generated by a liquid-displacing member of the cleaning device when the treatment unit is in a predefined cleaning position relative to the cleaning device.

The sanitizing device 400 is configured to disinfect the treatment unit of the personal care device. The sanitizing device 400 comprises a light source 410 configured to generate disinfecting light (e.g., UV light, or another type of disinfecting light such as blue light). The sanitizing device 400 also comprises a holding member 420 configured to hold the treatment unit of the personal care device in a predefined sanitizing position relative to the light source 410 such that the treatment unit is exposable to the disinfecting light. For example, as would be understood by the skilled person, the holding member 420 can comprise at least one of: a stand; a clamp; a bracket; and a magnetic attachment mechanism. The holding member 420 may be arranged in a casing of the sanitizing device 400, which may have a receiving space for receiving the personal care device or the treatment unit. The receiving space may be accessible by opening a closing member of the casing. Such aspects of a sanitizing device are well known to the skilled person and will not be described in further detail nor shown in the Figures. The sanitizing device 400 also comprises a processing unit 430.

The processing unit 430 is configured to: receive a first signal indicating a user action in relation to the sanitizing device 400; and receive a second signal indicating at least one of: a degree of debris on and/or in the treatment unit; and usage of the treatment unit since a last cleaning of the treatment unit by the cleaning device. The processing unit 430 is also configured to, when the first signal indicates the user action in relation to the sanitizing device 400 and the second signal indicates that the degree of debris and/or the usage of the treatment unit since the last cleaning exceeds a respective predefined threshold, generate an output signal relating to a recommended or required cleaning of the treatment unit by the cleaning device; and provide the output signal to a feedback member of the personal care system.

Referring now to Fig. 5, a block diagram of an embodiment of a sanitizing device 500 according to an aspect of the present invention is depicted. The sanitizing device 500 is substantially similar to the sanitizing device 400 of Fig. 4, although their differences will now be described.
In this embodiment, the sanitizing device 500 comprises a feedback member 540 to which the processing unit 430 of the sanitizing device 500 provides the output signal. This provides a convenient place for a user to receive a notification about a required or recommended cleaning of the treatment unit by the cleaning device of the personal care system in case the user intends to use the sanitizing device 500 to sanitize the treatment unit of the personal care device.

In this embodiment, the sanitizing device 500 also comprises a detector 550 configured to detect placement of the treatment unit of the personal care device in the predefined sanitizing position by the user. In this embodiment, the first signal is provided by the detector 550. For example, the detector 550 can comprise at least one of: an optical sensor; a proximity sensor; and a physical/mechanical switch.

In other embodiments, however, the detector 550 can be provided in the personal care device instead. In this case, the detector could further/instead comprise an inertial measurement unit in the personal care device, for example, which could help to indicate that the personal care device is at rest in the sanitizing device 500 or in a predefined orientation corresponding to the sanitizing position.

In this embodiment, the sanitizing device 500 also comprises a user-operable input element 560 (e.g., a button, a touch element, a switch, etc.) by which a user is enabled to activate the sanitizing device 500, and the first signal can also be provided by the user-operable input element.

Both the detector 550 and the user-operable input element 560 provide efficient and/or effective ways of providing the first signal indicating whether a user intends to use the sanitizing device 500. In other embodiments, the sanitizing device 500 can comprise only one of these components, or another component suitable to detect an intended use of the sanitizing device 500.

In this embodiment, the sanitizing device 500 also comprises a data storage unit 570 (e.g., a memory unit) configured to receive usage data about the treatment unit from the personal care device and cleaning data about the treatment unit from the cleaning device and/or the personal care device, and to store the received usage data and the received cleaning data. In this embodiment, the second signal indicates usage of the treatment unit since the last cleaning of the treatment unit by the cleaning device and is provided by the data storage unit based on the stored usage data and cleaning data.

Further, in this embodiment, the personal care device of the personal care system (of which the sanitizing device 500 is configured for use in) may comprise a main body and at least two different treatment units that can each be selectively coupled to the main body, i.e., similar to the personal care device 110 of the embodiment of the personal care system 300 of Fig. 3. Similar to the data storage unit 150 of the personal care system 300 of Fig. 3, the data storage unit 570 may be configured to store, for each of the different treatment units, received usage data and received cleaning data, and the processing unit 430 may be configured to: receive an identification signal indicating which of the different treatment units is identified in the sanitizing position; and receive, from the data storage unit 570 and for the identified treatment unit, the second signal indicating the usage of the identified treatment unit since a last cleaning of the identified treatment unit by the cleaning device of the personal care system. In other embodiments, the above functionality can be facilitated even if the data storage unit 570 is separate/external to the sanitizing device 500, e.g., in a user's smart device or in the cloud. Furthermore, in some embodiments, the sanitizing device 500 can be configured to: receive the personal care device including the main body and the treatment unit coupled to the main body; and generate the identification signal based on an output signal provided by an identification system of the personal care device which is configured to identify which of the different treatment units is actually coupled to the main body.

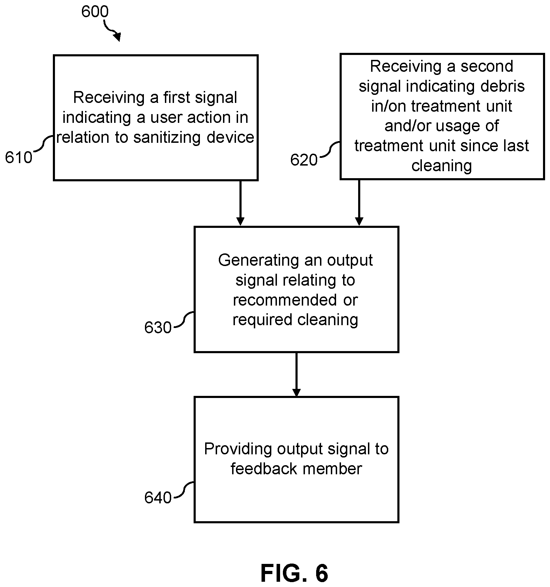

Referring now to Fig. 6, a flow diagram of an embodiment of a computer-implemented method 600 of operating a personal care system according to an aspect of the present invention is depicted. The personal care system comprises a personal care device, a sanitizing device, and a cleaning device. The personal care device comprises a treatment unit configured to treat a portion of a user's body. The sanitizing device is configured to disinfect the treatment unit by means of disinfecting light generated by a light source of the sanitizing device when the treatment unit is in a predefined sanitizing position relative to the light source. The cleaning device is configured to clean the treatment unit by means of a cleaning-liquid flow generated by a liquid-displacing member of the cleaning device when the treatment unit is in a predefined cleaning position relative to the cleaning device.

The computer-implemented method 600 comprises step 610 of receiving a first signal generated by the sanitizing device and indicating a user action in relation to the sanitizing device. Step 620 comprises receiving a second signal indicating at least one of: a degree of debris on and/or in the treatment unit; and usage of the treatment unit since a last cleaning of the treatment unit by the cleaning device.

Step 630 comprises generating an output signal relating to a recommended or required cleaning of the treatment unit by the cleaning device when the first signal indicates the user action in relation to the sanitizing device and the second signal indicates that the degree of debris and/or the usage of the treatment unit since the last cleaning exceeds a respective predefined threshold. Step 640 comprises generating an output signal relating to a recommended or required cleaning of the treatment unit by the cleaning device, and providing the output signal to a feedback member of the personal care system.

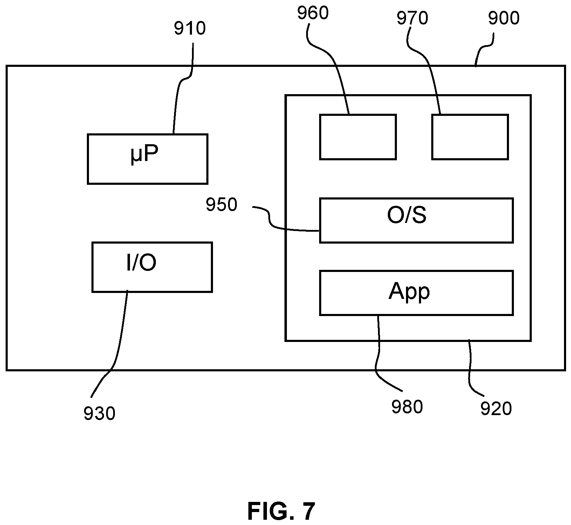

Fig. 7 illustrates an example of a computer 900 within which one or more parts of an embodiment may be employed. Various operations discussed above may utilize the capabilities of the computer. For example, one or more parts of a proposed personal care system may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor-based microprocessor (in the form of a microchip) or a microprocessor. For example, the processor 910 can be used to implement the method 600 of Fig. 6.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.
If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A personal care system (100) comprising a personal care device (110), a sanitizing device (120), a cleaning device (130), and a processing unit (140), wherein:
the personal care device (110) comprises a treatment unit (112) configured to treat a portion of a user's body;
the sanitizing device (120) is configured to disinfect the treatment unit by means of disinfecting light generated by a light source of the sanitizing device when the treatment unit is in a predefined sanitizing position relative to the light source; and
the cleaning device (130) is configured to clean the treatment unit by means of a cleaning-liquid flow generated by a liquid-displacing member of the cleaning device when the treatment unit is in a predefined cleaning position relative to the cleaning device;
and wherein the processing unit (140) is configured to:
receive a first signal generated by the sanitizing device (120) and indicating a user action in relation to the sanitizing device;
receive a second signal indicating at least one of: a degree of debris on and/or in the treatment unit; and usage of the treatment unit since a last cleaning of the treatment unit by the cleaning device;
when the first signal indicates the user action in relation to the sanitizing device and the second signal indicates that the degree of debris and/or the usage of the treatment unit since the last cleaning exceeds a respective predefined threshold, generate an output signal relating to a recommended or required cleaning of the treatment unit by the cleaning device; and
provide the output signal to a feedback member (114) of the personal care system.

2. The personal care system as claimed in claim 1, wherein the first signal indicates at least one of: placement of the treatment unit (112) in the predefined sanitizing position by the user; and activation of the sanitizing device (120) by the user.

3. The personal care system as claimed in claim 1 or 2, wherein the processing unit (140) is configured to, when the first signal indicates the user action in relation to the sanitizing device (120) and the second signal indicates that the degree of debris and/or the usage of the treatment unit (112) since the last cleaning exceeds the respective predefined threshold, prevent the activation of the light source of the sanitizing device.

4. The personal care system as claimed in any of claims 1-3, wherein the second signal is provided by a sensor (116) which is arranged in the personal care device (110) and configured to measure a parameter which is related to the degree of debris on and/or in the treatment unit (112).

5. The personal care system as claimed in any of the claims 1-3, wherein:
the personal care system comprises a data storage unit (150) configured to receive usage data about the treatment unit (112) from the personal care device (110) and cleaning data about the treatment unit from the cleaning device (130) and/or the personal care device, and to store the received usage data and the received cleaning data; and
the second signal indicates usage of the treatment unit since the last cleaning of the treatment unit by the cleaning device and is provided by the data storage unit based on the stored usage data and cleaning data.

6. The personal care system as claimed in claim 5, wherein the second signal indicates, with respect to the treatment unit (112), an accumulated treatment time and/or a number of treatment sessions performed since the last cleaning of the treatment unit by the cleaning device (130).

7. The personal care system as claimed in claim 5 or 6, wherein the data storage unit (150) is provided in the personal care device (110).

8. The personal care system as claimed in any of claims 5-7, wherein the personal care device (110) comprises:
a main body (111) and at least two different treatment units (112a, 112b) that can each be selectively coupled to the main body; and
an identification system (118) configured to identify which of the different treatment units is actually coupled to the main body;
wherein the data storage unit (150) is configured to store, for each of the different treatment units, received usage data and received cleaning data; and
wherein the processing unit (140) is configured to:
receive an identification signal from the sanitizing device indicating which of the different treatment units is identified in the sanitizing position; and
receive, from the data storage unit and for the identified treatment unit, the second signal indicating the usage of the treatment unit since a last cleaning of the identified treatment unit by the cleaning device (130).

9. The personal care system as claimed in claim 8, wherein the sanitizing device (120) is configured to receive the personal care device (110) including the main body (111) and the treatment unit (112a) coupled to the main body, and to generate the identification signal based on an output signal provided by the identification system (118) of the personal care device.

10. A sanitizing device (400) for use in a personal care system comprising the sanitizing device, a personal care device, and a cleaning device, wherein the personal care device comprises a treatment unit configured to treat a portion of a user's body, and wherein the cleaning device is configured to clean the treatment unit by means of a cleaning-liquid flow generated by a liquid-displacing member of the cleaning device when the treatment unit is in a predefined cleaning position relative to the cleaning device;
wherein the sanitizing device is configured to disinfect the treatment unit and comprises:
a light source (410) configured to generate disinfecting light;
a holding member (420) configured to hold the treatment unit in a predefined sanitizing position relative to the light source such that the treatment unit is exposable to the disinfecting light; and
a processing unit (430);
wherein the processing unit is configured to:
receive a first signal indicating a user action in relation to the sanitizing device;
receive a second signal indicating at least one of: a degree of debris on and/or in the treatment unit; and usage of the treatment unit since a last cleaning of the treatment unit by the cleaning device;
when the first signal indicates the user action in relation to the sanitizing device and the second signal indicates that the degree of debris and/or the usage of the treatment unit since the last cleaning exceeds a respective predefined threshold, generate an output signal relating to a recommended or required cleaning of the treatment unit by the cleaning device; and
provide the output signal to a feedback member of the personal care system.

11. The sanitizing device as claimed in claim 10, wherein the sanitizing device comprises the feedback member (540).

12. The sanitizing device as claimed in claim 10 or 11, further comprising:
a detector (550) configured to detect placement of the treatment unit in the predefined sanitizing position by the user, wherein the first signal is provided by the detector; and/or
a user-operable input element (560) by which a user is enabled to activate the sanitizing device, wherein the first signal is provided by the user-operable input element.

13. The sanitizing device as claimed in any of claims 10-12, further comprising a data storage unit (570) configured to receive usage data about the treatment unit from the personal care device and cleaning data about the treatment unit from the cleaning device and/or the personal care device, and to store the received usage data and the received cleaning data, wherein the second signal indicates usage of the treatment unit since the last cleaning of the treatment unit by the cleaning device and is provided by the data storage unit based on the stored usage data and cleaning data.

14. A computer-implemented method (600) of operating a personal care system comprising a personal care device, a sanitizing device, and a cleaning device, wherein:
the personal care device comprises a treatment unit configured to treat a portion of a user's body;
the sanitizing device is configured to disinfect the treatment unit by means of disinfecting light generated by a light source of the sanitizing device when the treatment unit is in a predefined sanitizing position relative to the light source; and
the cleaning device is configured to clean the treatment unit by means of a cleaning-liquid flow generated by a liquid-displacing member of the cleaning device when the treatment unit is in a predefined cleaning position relative to the cleaning device;
wherein the method (600) comprises:
receiving a first signal (610) generated by the sanitizing device and indicating a user action in relation to the sanitizing device;
receiving a second signal (620) indicating at least one of: a degree of debris on and/or in the treatment unit; and usage of the treatment unit since a last cleaning of the treatment unit by the cleaning device;
when the first signal indicates the user action in relation to the sanitizing device and the second signal indicates that the degree of debris and/or the usage of the treatment unit since the last cleaning exceeds a respective predefined threshold, generating an output signal (630) relating to a recommended or required cleaning of the treatment unit by the cleaning device; and
providing the output signal (640) to a feedback member of the personal care system.

15. A computer program comprising computer program code which is configured, when said program is run on a processing unit of a personal care system, to implement the method as claimed in claim 14.
